# EUROPEAN PATENT APPLICATION

(11) **EP 4 582 550 A1**
(43) Date of publication of application: **09.07.2025**
(21) Application number: 24749656.5
(22) Date of filing: 29.01.2024
(51) Int. Cl.: C12P 19/34, C12Q 1/6869, C12N 15/10, C12M 3/00

(54) **METHOD FOR TEMPLATE-FREE DE NOVO SYNTHESIS OF LONG-CHAIN NUCLEIC ACID, AND USE THEREOF**

(30) Priority: 31.01.2023 CN 202310046681; 17.04.2023 CN 202310408341
(71) Applicant: Shanghai Typing Bioscience Co., Ltd., Shanghai 200135 (CN)
(72) Inventor: XU, Cheng, Shanghai 200135 (CN); LIU, Yuanxiao, Shanghai 200135 (CN)
(74) Representative: Ullrich & Naumann PartG mbB
(86) International application number: PCT/CN2024/074510
(87) International publication number: WO 2024/160177

(57) **Abstract**

The present application relates to the fields of molecular biology and biotechnology, and in particular to a method for template-free de novo synthesis of a long-chain nucleic acid, comprising the following steps: S1, synthesis of double-stranded oligonucleotides; and S2, combination and ligation to obtain a target long-chain nucleic acid. The present application achieves continuous synthesis from single nucleotides to long-chain nucleic acids by means of the combination of S1 and S2, and has the advantages of no need for templates, high accuracy, low complexity and low cost.

## Description

### TECHNICAL FIELD

The present application relates to the fields of molecular biology and biotechnology, in particular to a method for template-free de novo synthesis of a long-chain nucleic acid, and a use thereof.

### BACKGROUND ART

At present, in the research and application of molecular biology, genetic engineering and synthetic biology, it is often necessary to synthesize large-fragment DNA sequences for detecting and studying their gene functions and other purposes. DNA synthesis methods mainly include a chemical synthesis method and an enzymatic assembly method.

The chemical synthesis method in related technologies is widely favored due to its mature technology and low cost, and allows for high-throughput production of DNAs or RNAs in chips in some embodiments. However, as the length of a synthetic template increases, errors accumulate gradually, a proportion of correct products sharply decreases, the cost of purification in the later stage rises, and the efficiency decreases. Therefore, an upper limit of sequences produced by this method in size is usually 200 bp.

The enzymatic assembly method in related technologies involves enzymatic ligation of oligonucleotides and polynucleotides of different sizes and sequences in a specific way to obtain larger molecules having desired target sequences. However, as the chain length increases or the number of ligated sequences increases, the accuracy and/or yield will decrease.

Moreover, a DNA fragment obtained by de novo synthesis has a limited length (below 600 bp). Longer genes or genomes (above 600 bp) need to be assembled through enzymatic/in-vivo assembly of oligonucleotide fragments, mainly in the form of LCR and PCA, but the synthesis efficiency and accuracy still cannot be guaranteed.

In summary, there is an urgent need to provide a method for template-free de novo synthesis of a long-chain nucleic acid with high accuracy, low complexity and low cost, and a use thereof.

### SUMMARY

To solve the above technical problem, the present application provides a method for template-free de novo synthesis of a long-chain nucleic acid, and a use thereof. This method achieves a highly accurate and continuous synthesis from single nucleotides to the long-chain nucleic acid through a combination of a specific enzymatic method and a ligase assembly method, without a need for templates.

In a first aspect, the present application provides a method for template-free de novo synthesis of a long-chain nucleic acid, including the following steps:
S1, synthesis of double-stranded oligonucleotides; and
S2, combination and ligation of the double-stranded oligonucleotides to obtain a target long-chain nucleic acid, wherein
the double-stranded oligonucleotides in step S1 are combined and ligated according to a sequence of the target long-chain nucleic acid; sequence numbers of the double-stranded oligonucleotides to be used are marked as 1, 2, 3, 4... N-1, N;
wherein N is a power of 2 to a power of n, i.e., 2ⁿ; and
a specific ligation method includes the following steps:
   (1) moving free double-stranded oligonucleotides marked as N respectively to a region where the double-stranded oligonucleotide marked as N-1 sequence is located, for a first ligation reaction to obtain N/2 first composite sequences;
      then, removing impurities by elution to remove a ligation system and unligated double-stranded oligonucleotide chains;
   (2) then, treating first composite sequences marked as N/2 with endonuclease to obtain free first composite sequences, moving the first free composite sequences respectively to a region where the first composite sequence marked as N/2-1 is located, for a second ligation reaction to obtain N/2² second composite sequences , and then removing impurities by elution; and
   (3) repeating the step (2) for several times until N/2", that is, one composite sequence is obtained, removing impurities by elution again after the reaction is completed, to remove the ligation system and unligated double-stranded oligonucleotide chains to obtain a fixed target long-chain nucleic acid.

Preferably, the double-stranded oligonucleotide marked as N-1 in step (1) is either a fixed nucleic acid chain or a free nucleic acid chain.

Preferably, the synthesis of double-stranded oligonucleotides in step S1 includes the following steps:
1) firstly, fixing a starting single-stranded oligonucleotide at a reaction site by biotin;
2) then, adding an amplification reaction system containing 3'-blocked dNTPs and a deblocking reaction system into the reaction site in sequence, and repeating said process for several times until fixed single-stranded oligonucleotides are obtained;
3) subsequently, treating the fixed single-stranded oligonucleotides with endonuclease, such that an extension chain is separated from the starting single-stranded oligonucleotide to obtain free oligonucleotide chains by template-free synthesis; and
4) finally, moving the free oligonucleotide chains to a region where complementary pairing chains are located, and performing a heating reaction to obtain fixed double-stranded oligonucleotides, the complementary pairing chains being the fixed single-stranded oligonucleotides in step 3).

Preferably, components and contents thereof of the amplification reaction system in step 2) are as follows:
the amplification reaction system contains 1.0-5.0 uM of TdT enzyme, 200 uM-500 uM of 3'-O-phosphate blocked dNTPs, 50-400 mM of a potassium cacodylate buffer, 20-50 mM of Tris, and 3-6 mM of CoCl₂; and
reaction conditions for the amplification reaction system applied in step 2) are as follows: a reaction volume is 0.1-50 ul, and incubation is carried out at 25-45°C for 5-30 min.

Preferably, components and contents thereof of the deblocking reaction system in step 2) are as follows:
the deblocking reaction system contains 75-120 mM of Tris-HCl at pH 6.5, 8-15 mM of MgCl₂, 5-8 mM of 2-mercaptoethanol, and one unit of T4 polynucleotide kinase; and
reaction conditions for the deblocking reaction system applied in step 2) are as follows: a reaction volume is 0.1-50 ul, and incubation is carried out at 25-45°C for 5-30 min.

Preferably, the step 3) includes the following steps:
treating the fixed single-stranded oligonucleotides with endonuclease first, such that the extension chain and the starting single-stranded oligonucleotide are free in lysis buffer for a lysis reaction; and after the lysis reaction is completed, transferring a lysis product and inactivating the endonuclease by heating, to obtain the free oligonucleotide chains by template-free synthesis.

Preferably, components and contents thereof of the lysis buffer, and lysis reaction conditions in step 3) are as follows:
the lysis buffer contains 40-60 mM of K-Ac, 15-25 mM of Tris-Ac, 8-15 mM of Mg-Ac, and 1-5 mM of DTT; and
the lysis reaction conditions are as follows: a reaction volume is 0.1-50 ul, and 1-100 U of endonuclease V is reacted at 25-45°C for 15-60 min.

Preferably, the step 4) includes the following steps:
moving the free oligonucleotide chains by template-free synthesis to the region where the complementary pairing chains are located, maintaining the region at 95°C for 3-5 min, and then cooling the region to 25°C at a constant speed within 15 min to obtain the fixed double-stranded oligonucleotides, the complementary pairing chains being the fixed single-stranded oligonucleotides in step 3).

In a second aspect, the present application provides a use of the method for template-free de novo synthesis of the long-chain nucleic acid in a synthesis of a DNA fragment and a mutation of a DNA sequence.

Preferably, an application environment is any one of a centrifuge tube, a microfluidic device, a digital microfluidic device, a microarray or a biochip.

In summary, the present application has the following beneficial effects.
The present application achieves continuous synthesis from single nucleotides to the long-chain nucleic acid by means of a combination of S1 and S2, and the synthesis method has advantages of no need for templates, high efficiency, high accuracy, low complexity and low cost, which are reflected in the following aspects.

The high efficiency lies in that: the fixed single-stranded oligonucleotide synthesized by the enzymatic method in step S1 can be formed into a double-stranded oligonucleotide containing sticky ends required for ligation after simple separation and annealing in step S21, which may directly enter into the subsequent assembly reaction, and the assembly reaction proceeds exponentially.
The high accuracy lies in that:
the fixed single-stranded oligonucleotides in step S1 are synthesized by the enzymatic method instead of chemical methods, such that the whole synthesis reaction system is maintained in a relatively stable environment (all in a biological enzyme system), which is conducive to the unification of reaction conditions and eliminates the interference of chemical reagents and reactions on the system; and
in the synthesis of oligonucleotide chains and double-stranded oligonucleotides assembly and ligation reaction in step S2, an elution step is further inserted between each two extension reactions, and the elution step can minimize the influence of impurities in the reaction system on an extension effect during each elongation reaction, and further greatly reduce the number of erroneous products during short-chain assembly due to the properties of DNA ligase.

The low complexity and low cost lies in that: common reagents and instruments can be used for all steps in this method, unlike complex customized equipment (e.g., gene chips and automated pipette arms) or large raw material libraries (e.g., oligonucleotide libraries) or templates required in other patents.

### BRIEF DESCRIPTION OF THE DRAWINGS

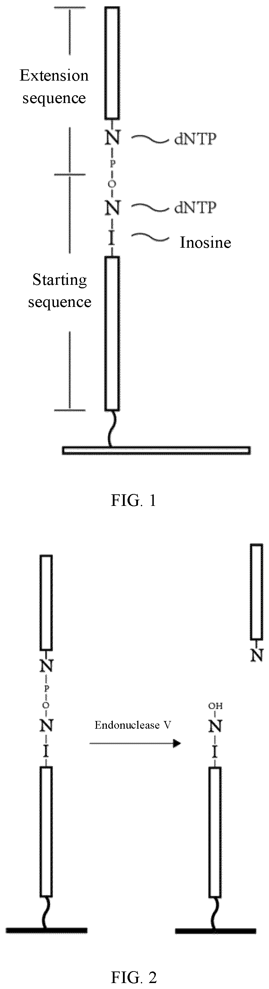

FIG. 1 is a schematic diagram of reactions in steps 1) and 2) for the synthesis of fixed single-stranded oligonucleotides in step S1.
FIG. 2 is a schematic diagram of a reaction in step 3) for a free oligonucleotide chain by template-free synthesis in step S1.

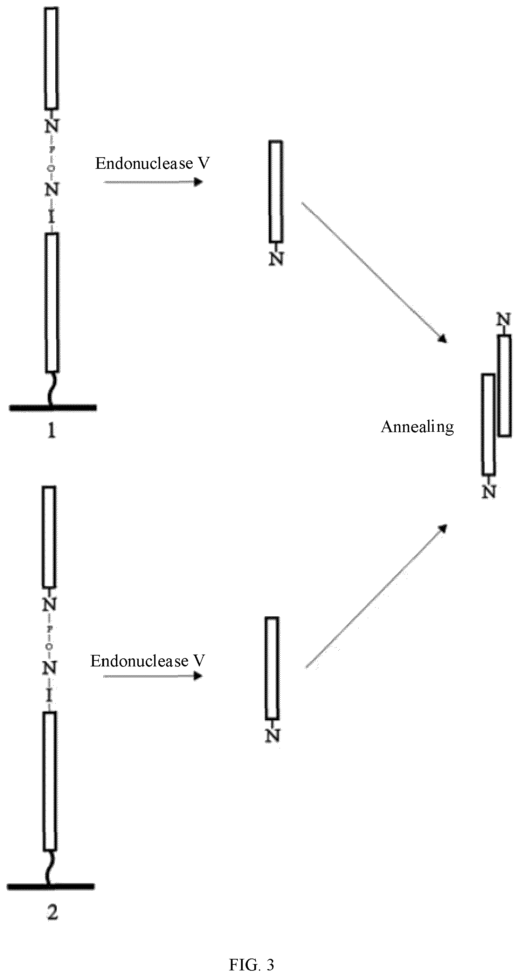

FIG. 3 is a schematic diagram of a reaction in step 4) for a free double-stranded oligonucleotide in step S1.

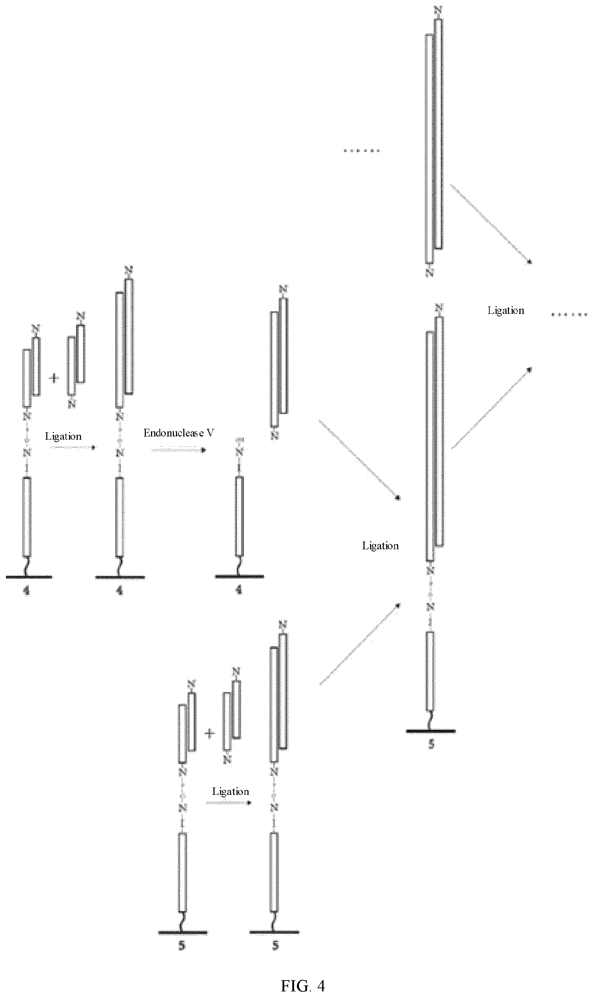

FIG. 4 is a schematic diagram of reactions in steps (1), (2) and (3) in step S2.
FIG. 5 is a schematic diagram of a sequence of a target long-chain nucleic acid to be synthesized.

### DETAILED DESCRIPTION

The present application is further explained below through specific examples and in conjunction with the drawings, but is not limited thereto. Various reaction systems used in the present application were shown in the following table:

| Types | Main components* |
|---|---|
| Amplification reaction system | 2.0 uM of TdT enzyme, 400 uM of 3'-O-phosphate blocked dNTPs, 100 mM of a potassium cacodylate buffer (pH 7.2), 25 mM of Tris, and 5 mM of CoCl₂ (pH 7.2) |
| Deblocking reaction system | 100 mM of Tris-HCl (pH 6.5), 10 mM of MgCl₂, 5 mM of 2-mercaptoethanol, and 5 U of T4 polynucleotide kinase |
| Lysis buffer | 50 mM of K-Ac, 20 mM of Tris-HCl, 10 mM of Mg-Ac, 1 mM of DTT dissolved in pH 7.9 |

| | |
|---|---|
| Note: the materials, reagents and instruments used in the specific examples of the present application, unless otherwise specified can be obtained commercially. | |

Materials: PCR tubes used - an inner wall of the tube was modified to contain biotin-streptomycin; and a starting single-stranded oligonucleotide with a length of 12 bp - 5' end of the starting single-stranded oligonucleotide was modified with streptavidin, a penultimate position at 3' end of the starting single-stranded oligonucleotide was hypoxanthine deoxyriboside (inosinate), and dNTPs-3' ends of the dNTPs were phosphorylated (3'-O-phosphate).

For sequenses of the single-stranded oligonucleotides to be used, see a.1-64 and b.1-64 respectively:
positive strands: 5'-3' ccatgaaaaggaactacattc 21 tggggctggacatcgggattacaagcgtg 29 gggtatgggattattgactatgaaacaag 29 ggacgtgatcgacgcaggcgtcagactgt 29 tcaaggaggccaacgtggaaaacaatgag 29 ggacggagaagcaagaggggagccaggcg 29 cctgaaacgacggagaaggcacagaatcc 29 agagggtgaagaaactgctgttcgattac 29 aacctgctgaccgaccattctgagctga 29 gtggaattaatccttatgaagccagggtga 29 aaggcctgagtcagaagctgtcagaggaa 29 gagttttccgcagctctgctgcacctggc 29 taagcgccgaggagtgcataacgtcaatg 29 aggtggaagaggacaccggcaacgagctg 29 tctacaaaggaacagatctcacgcaatag 29 caaagctctggaagagaagtatgtcgcag 29 agctacagctggaacggctgaagaaagat 29 ggcgaggtgagagggtcaattaataggtt 29 caagacaagcgactacgtcaaagaagcca 29 agcagctgctgaaagtgcagaaggcttac 29 caccagctggatcagagcttcatcgata 29 cttatatcgacctgctggagactcggaga 29 acctactatgagggaccaggagaagggagc 29 cccttcggatggaaagacatcaaggaatg 29 gtacgagatgctgatgggacattgcacc 29 tattttccagaagagctgagaagcgtcaa 29 gtacgcttataacgcagatctgtacaacgc 29 cctgaatgacctgaacaacctggtcatca 29 ccagggatgaaaacgagaaactggaata 29 ctatgagaagttccagatcatcgaaaacg 29 tgtttaagcagaagaaaaagcctacactga 29 aacagattgctaaggagatcctggtcaac 29 gaagaggacatcaagggctaccgggtgac 29 aagcactggaaaaccagagttcaccaat 29 ctgaaagtgtatcacgatattaaggacatc 29 acagcacggaaagaaatcattgagaacgc 29 cgaactgctggatcagattgctaagatc 29 ctgactatetacccagagttccgaggacatc 29 caggaagagctgactaacctgaacagcga 29 gctgacccaggaagagatcgaacagatt 29 agtaatctgaaggggtacaccggaacacac 29 aacctgtccctgaaagctatcaatctgat 29 tctggatgagctgtggcatacaaacgaca 29 atcagattgcaatctttaaccggctgaag 29 ctggtaccaaaaaaggtggacctgagtca 29 gcagaaagagatcccaaccacactggtgg 29 acgatttcattctgtcacccgtggtcaag 29 cggagcttcatccagagcatcaaagtgat 29 caacgccatcatcaagaagtacggcctg 29 cccaatgatatcattatcgagctggctag 29 ggagaagaacagcaaggacgcacagaagat 29 gatcaatgagatgcagaaacgaaaccggc 29 agaccaatgaacgcattgaagagattate 29 cgaactaccggpgaaagagaacgcaaagta 29 cctgattgaaaaaatcaagctgcacgata 29 tgcaggagggaaagtgtctgtattctctg 29 gaggecateccectggaggacetgetgaa 29 caatccattcaactacgaggtcgatcata 29 ttatccccagaagcgtgtccttcgacaa 29 ttcctttaacaacaaggtgctggtcaagca 29 ggaagagaactctaaaaagggcaatagga 29 ctcctttccagtacctgtctagttcaga 29 ttccaagatctcttacgaaacctttaaaaa 29 gcacattctgaatctggccaaggaaagg 29|
reverse complementary strands: 5'-3'. tccagccccagaatgtagttccttttcat 29 teccataccecacgettgtaatcccgatg 29 gatcacgtcccttgtttcatagtcaataa 29 gcctccttgaacagtctgacgcctgcgtc 29 ttctccgtccctcattgttttccacgttg 29 tcgtttcaggcgcctggctcccctcttgc 29 ttcaccctctggattctgtgccttctccg 29 tcagcaggttgtaatcgaacagcagtttc 29 attaattccactcagctcagaatggtcgg 29 ctcaggcctttcaccctggcttcataagg 29 cggaaaactcttcctctgacagcttctga 29 tcggcgccttagccaggtgcagcagagctg 29 tcttccacctcattgacgttatgcactcc 29 cctttgtagacagctcgttgccggtgtcc 29 cagagctttgctattgcgtgagatctgtt 29 agctgtagctctgcgacatacttctcttc 29 tcacctcgccatctttcttcagccgttcc 29 gcttgtcttgaacctattaattgaccctc 29 agcagctgcttggcttctttgacgtagtc 29 ccagctggtggtaagccttctgcactttc 29 gtcgatataagtatcgatgaagctctgat 29 tcatagtaggttctccgagtctccagcag 29 atccgaaggggctcccttctcctggtccc 29 catctcgtaccattccttgatgtctttcc 29 tctggaaaataggtgcaatgtcccatcag 29 tataagcgtacttgacgcttctcagctct 29 gtcattcagggcgttgtacagatctgcgt 29 tcatccctggtgatgaccaggttgttcag 29 acttctcatagtattccagtttctcgttt 29 ctgcttaaacacgttttcgatgatctgga 29 gcaatctgtttcagtgtaggctttttctt 29 tgtcctcttcgttgaccaggatctcctta 29 tccagtgcttgtcacccggtagcccttga 29 tacactttcagattggtgaactctggttt 29 tccgtgctgtgatgtccttaatatcgtga 29 cagcagttcggcgttctcaatgatttctt 29 tagatagtcaggatcttagcaatctgatc 29 gctcttcctggatgtcctcggaactctgg 29 ctgggtcagctcgctgttcaggttagtca 29 ttcagattactaatctgttcgatctcttc 29 gggacaggttgtgtgttccggtgtacccc 29 ctcatccagaatcagattgatagctttca 29 gcaatctgattgtcgtttgtatgccacag 29 ttggtaccagcttcagccggttaaagatt 29 ctctttctgctgactcaggtccacctttt 29 atgaaatcgtccaccagtgtggttgggat 29 tgaagctccgcttgaccacgggtgacaga 29 gatggcgttgatcactttgatgctctgga 29 atatcattgggcaggccgtacttcttgat 29 tgttcttctccctagccagctcgataatg 29 ctcattgatcatcttctgtgcgtccttgc 29 tcattggtctgccggtttcgtttctgcat 29 cggtagttcggataatctcttcaatgcgt 29 ttcaatcaggtactttgcgttctctttcc 29 ccctcctgcatatcgtgcagcttgatttt 29 ggatggcctccagagaatacagacacttt 29 gaatggattgttcagcaggtcctccaggg 29 ctggggataatatgatcgacctcgtagtt 29 tgttaaaggaattgtcgaaggacacgctt 29 gttctottcctgcttgaccagcaccttgt 29 tggaaaggagtcctattgccotttttaga 29 agatcttggaatctgaactagacaggtac 29 cagaatgtgctttttaaaggtttcgtaag 29 ctgatgcggccctttcctttggccagatt 29

### Example 1

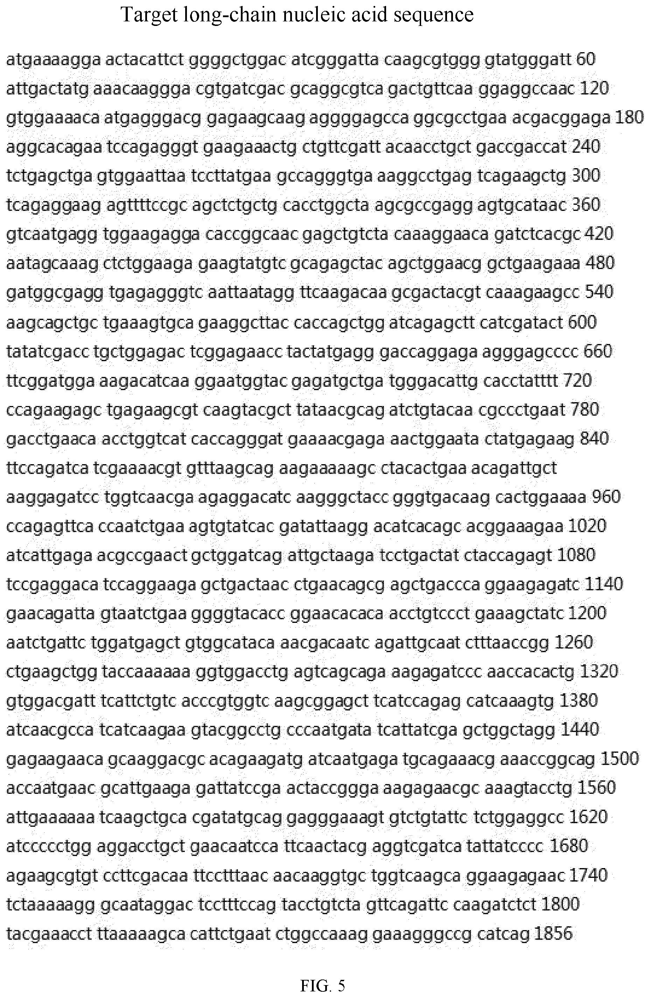

A method for template-free de novo synthesis of a long-chain nucleic acid was provided. A target long-chain nucleic acid to be synthesized had a length of 1856 bp, and a sequence as shown in FIG. 5, i.e., SEQ ID NO 1. The specific steps were as follows.
S1, synthesis of double-stranded oligonucleotides
   1) Firstly, 10 nmol of starting single-stranded oligonucleotide (starting sequence) was dissolved in 50 ul of ddH₂O then added to a biotin-modified PCR tube, the PCR tube was incubated for 30 min, such that the starting single-stranded oligonucleotide was fixed on a wall of the PCR tube; and the PCR tube was washed with 100 ul of ddH₂O twice, with five beats per blowing, to remove unfixed oligonucleotides.
   2) according to a sequence synthesis order of provided 128 oligonucleotide chains, the amplification reaction system containing 3'-O-phosphate blocked dNTPs was then added into 128 PCR tubes in sequence; each single nucleotide amplification reaction includes incubation with a reaction volume of 50 ul at 37°C for 5 min for amplification; each PCR tube was then washed with 100 ul of ddH₂O twice, with five beats per blowing, to remove the amplification reaction system;
      after each single nucleotide addition/amplification reaction, the deblocking reaction system was added to each washed PCR tube, and then incubated at 37°C for 1 min for a deblocking reaction; After the deblocking reaction, each PCR tube was washed with 100 ul of ddH₂O twice, with five beats per blowing, to remove the deblocking reaction system; and
      finally, repeating the above step 2) according to the sequence synthesis order of the oligonucleotide chains until the ligation and deprotection of the last nucleotide in the fixed single-stranded oligonucleotides were completed to obtain reverse complementary single-stranded oligonucleotides fixed on the wall of the PCR tube.
   3) The reverse complementary single-stranded oligonucleotides fixed on the wall of the PCR tube were then treated with endonuclease V, so that an extension chain and the starting single-stranded oligonucleotide were free in lysis buffer, and a lysis reaction was carried out with 100 U of endonuclease V with a reaction volume of 50 ul at 37°C for 15 min; and after the lysis reaction was completed, the buffer was transferred to a new tube, heated to 65°C and maintained for 10 min to inactivate endonuclease V, thereby obtaining free oligonucleotide chains by template-free synthesis.
   4) Finally, the free oligonucleotide chains by template-free synthesis were respectively transferred to a tube where complementary pairing chains were located, the tube was maintained at 95°C for 3 min, and then cooled to 25°C at a constant speed within 15 min to obtain 64 double-stranded oligonucleotides for the synthesis of SEQ ID NO 1, wherein
      the complementary pairing chains are positive-stranded oligonucleotides in step S1, the positive-stranded oligonucleotides in odd-numbered tubes were still fixed on the walls of the tubes, and the positive-stranded oligonucleotides in even-numbered tubes were in a free state.
S2, combination and ligation of the double-stranded oligonucleotides to obtain a target long-chain nucleic acid.

Free double-stranded oligonucleotides obtained in S21 were combined and ligated according to a sequence of the target long-chain nucleic acid; and the used PCR tubes for the double-stranded oligonucleotides were marked as 1, 2, 3,......, 63, 64; and a specific ligation method included the following steps:
(1) firstly, the double-stranded oligonucleotides marked as even numbers (set as N) were respectively moved to a tube where the double-stranded oligonucleotide marked as N-1 was located, and a ligation reaction was carried out by incubating with T4 ligase at room temperature for 60 min to obtain 32 tubes of extended composite sequences, which were renumbered as 1, 2, 3,......, 31, 32; each PCR tube was washed with 100 ul of ddH₂O twice, with five beats per blowing, to remove a ligation system and unligated double-stranded oligonucleotide chains;
(2) then, composite sequences marked as even numbers (set as N/2) were treated again with endonuclease V, such that the composite sequences marked as even numbers were free to obtain free composite sequences; the free composite sequences were then moved respectively to a tube where the composite sequence of N/2-1 was located; a ligation reaction was carried out by incubating with T4 ligase at room temperature for 60 min to obtain 16 tubes of extended composite sequences which were renumbered as 1, 2, 3,......, 15, 16; and similarly, each PCR tube was washed with 100 µl of ddH₂O twice, with five beats per blowing, to removethe ligation system and unligated double-stranded oligonucleotide chains;
(3) the operation in step (2) was repeated; after a total of 6 rounds of ligation (64 tubes-32 tubes-16 tubes-8 tubes-4 tubes-2 tubes-1 tube) were completed, the target long-chain nucleic acid, ultimately fixed to the wall of the PCR tube, can be obtained; and
similarly, the PCR tube was washed with 100 µl of ddH₂O twice, with five beats per blowing, to remove the ligation system and unligated double-stranded oligonucleotide chains.

Finally, the target long-chain nucleic acid fixed on the wall of each PCR tube was treated with endonuclease V and then free in buffer for subsequent detection. The specific detection step was as follows:
the free target long-chain nucleic acid was subjected to agarose gel electrophoresis, and then compared the band size of the target long-chain nucleic acid with that of the marker.

### Comparative Example 1

A method for template-free de novo synthesis of a long-chain nucleic acid was provided, which was different from Example 1 in that the synthesis method to be used is a PCA (Polymerase Cycling Assembly) method.

In the PCA method, a pair of nucleic acid chains that were complementary end to end was used for PCR amplification to obtain full-length double-stranded nucleic acids. For each amplification, a pair of primers that were complementary to a first end of the existing sequence were required as an extension sequence. Due to the nature of the PCR, complementary regions of the oligonucleotide sequences required were longer (>20 bp), and thus the oligonucleotide sequences required were also longer (>40 bp). In this example, the oligonucleotide sequences used were 45 bp, with a complementary region of 22 bp.

The specific synthesis method was as follows:
1) firstly, the oligonucleotide sequences including 1 positive strand and 80 reverse complementary amplification strands were synthesized;
2) the positive strand and reverse complementary amplification strands (including the complementary regions) were added to a PCR reaction system (100 µl) at the same time, wherein a final concentration of each strand in the reaction system was 0.1 µM, and the PCR reaction system was commercially formulated; the PCR reaction conditions were as follows: denaturation at 95°C for 5 min, then annealing at 50°C for 15 seconds, and extension at 72°C for 120 seconds; annealing and extension reactions were repeated for 30 times; and
3) after the reaction was completed, an amplification product was subjected to electrophoresis in agarose gel and band sizes of the amplification product were observed.

Experimental results: No target sequence-length fragment was observed in the agarose gel, indicating synthesis failure.
As can be seen from Example 1 and Comparative Example 1, the present application achieves continuous synthesis from single nucleotides to the long-chain nucleic acid by means of the combination of S1 and S2, and the synthesis method had the advantages of no need for templates, high efficiency, high accuracy, low complexity and low cost.
Obviously, Comparative Example 1 cannot possess the aforementioned advantages. The reasons were analyzed as follows.
The PCA method involved multiple DNA polymerization reactions, and a synthesis error rate of polymerase would affect the accuracy of the target nucleic acid chain; short chains may also mismatch during denaturation and annealing, and such mismatch would be ignored by DNA polymerase to increase the synthesis error rate. Since the PCA method was essentially a PCR reaction, accurate temperature control and repeated temperature changes were required during the reaction process. Such reaction conditions would also affect the efficiency of long-chain synthesis.

### Comparative Example 2

A method for template-free de novo synthesis of a long-chain nucleic acid was provided, which was different from Example 1 in that the synthesis method to be used was a Gibson assembly method.

The Gibson assembly method used three enzymes-DNA exonuclease, DNA polymerase, and DNA ligase-as tools to ligate a plurality of blunt-ended double-stranded DNAs containing complementary regions into a single strand. The DNA exonuclease was used to generates sticky ends, the DNA polymerase was used to complement missing bases at the ends after complementary pairing (the DNA exonuclease can remove excessive bases), and the DNA ligase was used to ligate two strands.

The specific method was as follows:
1) a total of 124 polynucleotide sequences, including 62 positive-stranded polynucleotide sequences and 62 negative-stranded polynucleotide sequences, were synthesized first and numbered from 1 to 62 in sequence; the positive-stranded and negative-stranded polynucleotide sequences with the same numbers were denatured and annealed at a final concentration of 20 µM in a 50 ul system to form complementary double-stranded nucleic acids; the sequence numbers of the products remained unchanged; and denaturation and annealing conditions were as follows: maintaining at 95°C for 5 min, and cooling at 25°C within 30 min;
2) the above-mentioned annealed double-stranded nucleic acids were subjected to a Gibson assembly reaction, all double-stranded nucleic acids were added to a 100 ul reaction system to assemble at a final concentration of 0.2 µM, and the reaction was carried out using a commercial Gibson assembly kit; and
3) after the reaction was completed, an amplification product was subjected to electrophoresis in agarose gel and band sizes of the amplification product were observed.

Experimental results: No target sequence-length fragment was observed in the agarose gel, indicating synthesis failure.

As can be seen from Example 1 and Comparative Example 2, the present application achieves continuous synthesis from single nucleotides to the long-chain nucleic acid by means of the combination of S1 and S2, and the synthesis method had the advantages of no need for templates, high efficiency, high accuracy, low complexity and low cost.
Obviously, Comparative Example 2 cannot possess the aforementioned advantages. The reasons were analyzed as follows.
Due to the uncontrollable nature of exonuclease, single-stranded nucleic acids that cannot be complemented by polymerases in time would be produced during the reaction, which affected the ligation efficiency, so the effect of this method depended on concentrations of the three enzymes and proportions of the buffer. In addition, when the ligated strand was too short, the DNA exonuclease would directly cut off the nucleic acid chains, so that the DNA polymerase failed to function. Therefore, this method was only suitable for the ligation of long-chain nucleic acids.

### Comparative Example 3

A method for template-free de novo synthesis of a long-chain nucleic acid was provided, which was different from Example 1 in that the synthesis method to be used was an LCR (Ligation Chain Reaction) method.

In the LCR method, with the help of a single-strand bridging oligonucleotide , 5' and 3' ends of two nucleic acid chains were joined into a single chain by DNA ligase, wherein the bridging oligonucleotide was complementarily paired with 5' and 3' ends of chains to be ligated, respectively.

The specific method was as follows:
1) 62 positive-stranded oligonucleotides and 61 bridging oligonucleotides were synthesized first, each bridging oligonucleotide corresponding to a complementary ligation reaction of two positive strands; all strands were added to a reaction system of 100 µl, wherein a final concentration of each strand was 0.1 µM; and the reaction conditions were as follows: maintaining at 95°C for 5 min, and then cooling to 25°C for DNA ligation for 30 min; a commercially available ligase was used;
2) after the reaction was completed, 5 µl of amplification product was added to a PCR reaction system, and amplification primers were added at the same time, and a target sequence was obtained after 30 PCR cycles; and
3) a PCR product was subjected to electrophoresis in agarose gel and band sizes of the PCR product were observed.

Experimental results: No target sequence-length fragment was observed in the agarose gel, indicating synthesis failure.

As can be seen from Example 1 and Comparative Example 3, the present application achieves continuous synthesis from single nucleotides to the long-chain nucleic acid by means of the combination of S1 and S2, and the synthesis method had the advantages of no need for templates, high efficiency, high accuracy, low complexity and low cost.
Obviously, Comparative Example 3 cannot possess the aforementioned advantages. The reasons were analyzed as follows.
The LCR method required the provision of double-stranded nucleic acids with sticky ends prior to ligation (the double-stranded nucleic acids in the present application were formed by annealing the synthesized oligonucleotides). Due to the specificity of the DNA ligase, the ligation reaction cannot occur when the sticky ends of the provided double-stranded nucleic acids were missing or mismatched and thus the ends were not aligned, thereby significantly reducing the number of false ligation products.

The specific examples are merely an explanation of the present application and not for limiting the present application. Those skilled in the art may make modifications, without creative contribution, to the examples as needed after reading this specification. Any of the modifications made within the scope of the claims of the present application shall be protected by the Patent Law.

## Claims

1. A method for template-free de novo synthesis of a long-chain nucleic acid, **characterized by** comprising the following steps:
S1, synthesis of double-stranded oligonucleotides; and
S2, combination and ligation of the double-stranded oligonucleotides to obtain a target long-chain nucleic acid, wherein
the double-stranded oligonucleotides in step S1 are combined and ligated according to a sequence of the target long-chain nucleic acid; sequence numbers of the double-stranded oligonucleotides to be used are marked as 1, 2, 3, 4... N-1, N;
wherein N is a power of 2 to a power of n, i.e., 2ⁿ; and
a specific ligation method comprises the following steps:
(1) moving free double-stranded oligonucleotides marked as N respectively to a region where the double-stranded oligonucleotide marked as N-1 is located, for a first ligation reaction to obtain N/2 first composite sequences;
then, removing impurities by elution to remove a ligation system and unligated double-stranded oligonucleotide chains;
(2) then, treating first composite sequences marked as N/2 with endonuclease to obtain free first composite sequences, moving the first free composite sequences respectively to a region where the first composite sequence marked as N/2-1 is located, for a second ligation reaction to obtain N/2² second composite sequences, and then removing impurities by elution; and
(3) repeating the step (2) for several times until N/2ⁿ, that is, one composite sequence is obtained, removing impurities by elution again after the reaction is completed, to remove the ligation system and unligated double-stranded oligonucleotide chains to obtain a fixed target long-chain nucleic acid.

2. The method for template-free de novo synthesis of the long-chain nucleic acid according to claim 1, **characterized in that**, the double-stranded oligonucleotide marked as N-1 in step (1) is either a fixed nucleic acid chain or a free nucleic acid chain.

3. The method for template-free de novo synthesis of the long-chain nucleic acid according to claim 1, **characterized in that**, the synthesis of double-stranded oligonucleotides in step S1 comprises the following steps:
1) firstly, fixing a starting single-stranded oligonucleotide at a reaction site by biotin;
2) then, adding an amplification reaction system containing 3'-blocked dNTPs and a deblocking reaction system into the reaction site in sequence, and repeating said process for several times until fixed single-stranded oligonucleotides are obtained;
3) subsequently, treating the fixed single-stranded oligonucleotides with endonuclease, such that an extension chain is separated from the starting single-stranded oligonucleotide to obtain free oligonucleotide chains for template-free synthesis; and
4) finally, moving the free oligonucleotide chains to a region where complementary pairing chains are located, and performing a heating reaction to obtain fixed double-stranded oligonucleotides, the complementary pairing chains being the fixed single-stranded oligonucleotides in step 3).

4. The method for template-free de novo synthesis of the long-chain nucleic acid according to claim 3, **characterized in that**, components and contents thereof of the amplification reaction system in step 2) are as follows:
the amplification reaction system contains 1.0-5.0 uM of TdT enzyme, 200 uM-500 uM of 3'-O-phosphate blocked dNTPs, 50-400 mM of a potassium cacodylate buffer, 20-50 mM of Tris, and 3-6 mM of CoCl₂; and
reaction conditions for the amplification reaction system applied in step 2) are as follows: a reaction volume is 0.1-50 ul, and incubation is carried out at 25-45°C for 5-30 min.

5. The method for template-free de novo synthesis of the long-chain nucleic acid according to claim 3, **characterized in that**, components and contents thereof of the deblocking reaction system in step 2) are as follows:
the deblocking reaction system contains 75-120 mM of Tris-HCl at pH 6.5, 8-15 mM of MgCl₂, 5-8 mM of 2-mercaptoethanol, and one unit of T4 polynucleotide kinase; and
reaction conditions for the deblocking reaction system applied in step 2) are as follows: a reaction volume is 0.1-50 ul, and incubation is carried out at 25-45°C for 5-30 min.

6. The method for template-free de novo synthesis of the long-chain nucleic acid according to claim 3, **characterized in that**, the step 3) comprises the following steps:
treating the fixed single-stranded oligonucleotides with endonuclease first, such that the extension chain and the starting single-stranded oligonucleotide are free in lysis buffer for a lysis reaction; and after the lysis reaction is completed, transferring a lysis product and inactivating the endonuclease by heating, to obtain the free oligonucleotide chains for template-free synthesis.

7. The method for template-free de novo synthesis of the long-chain nucleic acid according to claim 6, **characterized in that**, components and contents thereof of the lysis buffer, and lysis reaction conditions in step 3) are as follows:
the lysis buffer contains 40-60 mM of K-Ac, 15-25 mM of Tris-Ac, 8-15 mM of Mg-Ac, and 1-5 mM of DTT; and
the lysis reaction conditions are as follows: a reaction volume is 0.1-50 ul, and 1-100 U of endonuclease V is reacted at 25-45°C for 15-60 min.

8. The method for template-free de novo synthesis of the long-chain nucleic acid according to claim 3, **characterized in that**, the step 4) comprises the following steps:
moving the free oligonucleotide chains for template-free synthesis to the region where the complementary pairing chains are located, maintaining the region at 95°C for 3-5 min, and then cooling the region to 25°C at a constant speed within 15 min to obtain the fixed double-stranded oligonucleotides, the complementary pairing chains being the fixed single-stranded oligonucleotides in step 3).

9. Use of the method for template-free de novo synthesis of the long-chain nucleic acid according to any one of claims 1 to 8 in a synthesis of a DNA fragment and a mutation of a DNA sequence.

10. Use of the method for template-free de novo synthesis of the long-chain nucleic acid according to claim 9, **characterized in that**, an application environment is any one of a centrifuge tube, a microfluidic device, a digital microfluidic device, a microarray or a biochip.
